# EUROPÄISCHE PATENTSCHRIFT

(11) **EP 1 203 596 B1**
(45) Veröffentlichungstag und Bekanntmachung des Hinweises auf die Patenterteilung: **23.11.2005**
(21) Anmeldenummer: 01250365.2
(22) Anmeldetag: 18.10.2001
(51) Int. Cl.: A61N 1/32, A61N 1/20, A61N 1/28, A61B 18/18, A61F 2/02, A61F 2/04, A61F 2/06, A61N 2/00, A61N 2/02, A61N 5/02, A61N 5/00, A61N 1/372, A61N 1/375, A61N 1/378, A61N 1/40

(54) **Vorrichtung zum Beeinflussen von Zellproliferationsmechanismen in Gefässen des menschlichen oder tierischen Körpers**
Apparatus for promoting cell growth of human or animal tissues
Dispositif pour accélérer la prolifération des tissus du corps humain ou animal

(30) Priorität: 03.11.2000 DE 10055686
(43) Veröffentlichungstag der Anmeldung: 08.05.2002
(73) Patentinhaber: BIOTRONIK Mess- und Therapiegeräte GmbH & Co Ingenieurbüro Berlin, 12359 Berlin (DE)
(72) Erfinder: Stoll, Hans-Peter, 66386 St. Ingbert (DE); Schmiedl, Robert, 91058 Erlangen (DE)
(74) Vertreter: Eisenführ, Speiser & Partner

(56) Entgegenhaltungen:
- EP-A- 1 036 574
- WO-A-00/13585
- US-A- 5 078 736
- W. SONNTAG, H. DERTINGER: "Response of cytosolic calcium, cyclic AMP, and cyclic GMP in dimethylsulfoxide-differentiated HL-60 cells to modulated low frequency electric currents" BIOELECTROMAGNETICS, [Online] Bd. 19, 1998, Seiten 452-458, XP002197569 Gefunden im Internet: <URL:http://www.ncbi.nlm.nih.gov/entrez/qu ery.fcgi> [gefunden am 2002-04-26]
- CIRO INDOLFI, EUGENIO STABILE, CINZIA PERRINO, MASSIMO CHIARIELLO: "Mechanisms of restenosis after angioplasty and appoach to therapy (Review)" INTERNATIONAL JOURNAL OF MOLECULAR MEDICINE, [Online] Bd. 2, 1998, Seiten 143-148, XP002197570 Gefunden im Internet: <URL:http://www.ncbi.nlm.nih.gov/entrez/qu ery.fcgi> [gefunden am 2002-04-26]
- G. NINDL, W.X. BALCAVAGE, D.N. VESPER, J.A. SWEZ, B.J. WETZEL, J.K. CHAMBERLAIM, M.T. FOX: "Experiments showing that electromagnetic fields can be used to treat inflammatory diseases" BIOMEDICAL SCIENCES INSTRUMENTATION, [Online] Bd. 36, 16. April 2000 (2000-04-16), Seiten 7-13, XP002197571 Gefunden im Internet: <URL:http://www.ncbi.nlm.nih.gov/entrez/qu ery.fcgi> [gefunden am 2002-04-26]
- E. BETZ: "Migration ud Proliferation von glatten Muskelzellen in der Gefässwand" ARZNEIMITTELFORSCHUNG, [Online] Bd. 40, Nr. 3A, März 1990 (1990-03), Seiten 362-365, XP001068929 Gefunden im Internet: <URL:http://www.ncbi.nlm.nih.gov/entrez/qu ery.fcgi> [gefunden am 2002-04-26]

## Beschreibung

Die vorliegende Erfindung betrifft eine Vorrichtung zum Beeinflussen von Zellproliferationsmechanismen in Gefäßen, insbesondere Blutgefäßen, des menschlichen oder tierischen Körpers. Sie betrifft weiterhin ein Implantat zum Einsetzen in ein Gefäß des menschlichen oder tierischen Körpers, insbesondere einen Stent.

Zellproliferationsmechanismen in der Gefäßwand spielen regelmäßig eine wichtige Rolle im Zusammenhang mit der Behandlung von Defekten an Gefäßen des menschlichen oder tierischen Körpers. Die Zellproliferation kann zum einen Ursache für einen solchen Defekt sein, wie dies beispielsweise bei Stenosen in Blutgefäßen der Fall ist. Vermindertes bzw. langsames Zellwachstum kann aber auch beispielsweise die Ursache für eine unbefriedigend langsame Heilung von Defekten eines Gefäßes sein.

Eine Anzahl unterschiedlichster Erkrankungen kann zu so genannten Stenosen, d. h. Verengungen in Gefäßen des Körpers, mit zum Teil zu schwerwiegenden oder sogar tödlichen Konsequenzen führen. Hierbei sind häufig die Blutgefäße des Körpers betroffen. So stellt beispielsweise die Arteriosklerose mit den mit ihr einhergehenden Gefäßverengungen die wichtigste und häufigste krankhafte Veränderung der Arterien dar, die schwerwiegendste Folgen nach sich ziehen kann.

Zur Behandlung oder Prophylaxe solcher Stenosen werden unterschiedliche Wege eingeschlagen. So werden beispielsweise zur Stenose-Prophylaxe oder zur Behandlung noch wenig weit fortgeschrittener Verengungen medikamentöse Behandlungen eingesetzt sowie dem Patienten entsprechende Diät verschrieben, während weit fortgeschrittene Stenosen in der Regel operativ behandelt werden. Hierbei werden die betroffenen Stellen des Gefäßes meist mittels eines Ballonkatheters aufgeweitet. Bei dieser Ballondilatation ist es häufig nötig, einen so genannten Stent in das Gefäß einzusetzen, um dieses aufgeweitet zu halten.

Um nach einer Behandlung des Gefäßes so genannte Restenosen zu verhindern, wurden neben medikamentösen Behandlungen und entsprechender Umstellung der Ernährung des Patienten auch Stents vorgeschlagen, die auf ihrer dem Gefäß zugewandten Seite mit Geweben oder dergleichen bedeckt sind, um das erneute Verengen des Gefäßes infolge des durch Zellproliferation, d. h. unkontrollierte Wucherung der Gefäßwandzellen, bewirkten Einwachsens der Gefäßwand in die Gefäßbahn zu verhindern.

Die vorgeschlagenen Methoden weisen unterschiedliche Nachteile auf. So kann die Umstellung der Ernährung oft nur unterstützend wirken, während die medikamentöse Behandlung zwar meist recht erfolgreich eingesetzt werden kann, je nach Patient aber auch unterschiedlichste Nebenwirkungen hervorrufen kann. Die invasive Behandlung mit entsprechend ausgebildeten, die Gefäßwand komplett abdeckenden Stents ist zum einen relativ aufwändig, zum anderen ist nicht abzusehen, inwieweit die ungebremste Zellproliferation unter der Abdeckung zu einer möglicherweise bedrohlichen Belastung für das Gefäß wird.

Zur Beschleunigung der Heilung von Defekten eines Gefäßes, beispielsweise zur beschleunigten Wundheilung nach einem chirurgischen Eingriff, werden in der Regel nur medikamentöse Behandlungen mit den oben bereits geschilderten Nachteilen vorgeschlagen.

Aus dem Artikel von C. Indolfi, E. Stabile, C. Perrino und M. Chiariello: "Mechanisms of restenosis after angioplasty and approach to therapy (Review)", Intemational Journal of Molecular Medicine, Bd. 2, 1998, Seiten 143 - 148, ist der Zusammenhang zwischen cAMP-Aktivität in der Zelle und dem Mechanismus der Proliferation bekannt. Die Autoren schlagen vor, nach der Implantation eines Stents spezifische die cAMP-Aktivität steuernde Arzneimittel zur Restenose-Prophylaxe einzusetzen.

W. Sonntag und H. Dertinger beschreiben in dem Artikel: "Response of Cytosolic Calcium, Cyclic AMP, and Cyclic GMP in Dimethylsulfoxide-Differentiated HL-60 Cells to Modulated Low Frequency Electric Currents", BIOELECTROMAGNETICS, Bd. 19, 1998, Seiten 452 - 458, den Einfluss niederfrequenter modulierter elektrischer Ströme auf die cAMP-Aktivität.

Dervorliegenden Erfindung liegt daher die Aufgabe zu Grunde, eine Vorrichtung zur Beeinflussung von Zellproliferationsmechanismen in Gefäßen zur Verfügung zu stellen, welche die oben beschriebenen Nachteile zumindest in geringerem Maße aufweist und welche insbesondere mit geringem Aufwand und möglichst wenig Nebenwirkungen für den Patienten einsetzbar ist.

Diese Aufgabe wird ausgehend von einer Vorrichtung gemäß dem Oberbegriff des Anspruchs 1 durch die im kennzeichnenden Teil des Anspruchs 1 angegebenen Merkmale gelöst. Weiterhin wird sie ausgehend von einem Implantat gemäß den Oberbegriff des Anspruchs 8 durch die im kennzeichnenden Teile des Anspruchs 8 angegebenen Merkmale gelöst.

Die vorliegende Erfindung schließt die technische Lehre ein, dass man durch eine geeignete Vorrichtung zur Elektrostimulation der Zellen der Gefäßwand mit Stimulationsströme mit geringem Aufwand und geringen Nebenwirkungen für den Patienten eine vorteilhafte Beeinflussung der Zellproliferationsmechanismen in der Gefäßwand erzielen kann. Dabei kann je nach Anwendungsfall eine zumindest teilweise Unterdrückung von Zellproliferationsmechanismen erzielt werden, wie dies beispielsweise zur Verhinderung bzw. Verlangsamung von Stenosen erforderlich ist. Ebenso kann aber auch eine Anregung der Zellproliferation bzw. des Zellwachstums erzielt werden, wie dies beispielsweise zur Beschleunigung der Wundheilung oder zur Stabilisierung von solchen Gefäßen von Vorteil ist.

Die Elektrostimulation zur Unterdrückung von Zellproliferationsmechanismen ist bisher nur im Zusammenhang mit der Behandlung von Psoriasis (Schuppenflechte) für Hautzellen bekannt, die naturgemäß an der Körperoberfläche liegen und daher für eine einfache Stimulation mit entsprechenden Stimulationsströmen durch direkte Kontaktierung ohne weiteres zugänglich sind (vgl. Spektrum der Wissenschaft, Monatsspektrum, April 2000, S. 15 bis 17, Spektrum der Wissenschaft Verlagsgesellschaft mbH, Heidelberg, DE).

Es hat sich gezeigt, dass die Vorteile der Elektrostimulation auch im Bereich von Gefäßen, d. h. im nicht ohne weiteres zugänglichen Körperinneren mit geringem Aufwand und ohne Nebenwirkungen für den Patienten mit einfachen Mitteln erzielbar sind, wenn erfindungsgemäß eine Erregereinrichtung zur Bewirkung von Stimulationsströmen in einem zu behandelnden Bereich des Gefäßes vorgesehen ist. Die Erregereinrichtung ist dabei erfindungsgemäß zur Bewirkung von niedrigfrequenten Stimulationsströmen ausgebildet, deren Frequenz im Bereich der Frequenzen liegt, bei denen durch ihre biologisch wirksame Information die Ausschüttung von zyklischem Adenosinmonophosphat (cAMP) gehemmt oder angeregt wird. Da die biologisch wirksame Information dabei im Frequenz- und/oder Modulationsmuster der Stimulationsströme liegt, kann dieselbe Wirkung erreicht werden, wenn die Erregereinrichtung erfindungsgemäß zur Bewirkung von niedrigfrequent modulierten Stimulationsströmen ausgebildet ist, wobei dann die Modulationsfrequenz im Bereich der Frequenzen liegt, bei denen die Ausschüttung von die Zellproliferation steuernden sekundären Botenstoffen, insbesondere von zyklischem Adenosinmonophosphat (cAMP), in den Zellen des Gefäßes beeinflusst, d. h. gehemmt oder angeregt wird. Ebenso können entsprechend niedrigfrequente Stimulationsströme zusätzlich noch entsprechend niedrigfrequent moduliert sein. Die betreffende Frequenz kann von Zelltyp zu Zelltyp abweichen. Hierbei verringert beispielsweise eine höhere Konzentration von zyklischem Adenosinmonophosphat die Teilungsaktivität der Zellen, während diese durch eine verringerte Konzentration erhöht wird.

Erfindungsgemäß ist die Erregereinrichtung zum berührungslosen Einbringen der Stimulationsenergie, die zur Bewirkung der Stimulationsströme dient, in ein Implantat ausgebildet, das im zu behandelnden Bereich des Gefäßes angeordnet ist. Bei dem Implantat kann es sich insbesondere um einen Stent handeln. Diese Varianten sind speziell dann von Vorteil, wenn an der zu behandelnden Stelle des Gefäßes bereits ein solches Implantat, also beispielsweise ein Stent, angeordnet ist bzw. angeordnet werden muss. Ein solcher Stent kann z. B. notwendig sein, um das Gefäß in einer aufgeweiteten Position zu halten, welche das Gefäß von sich aus nicht bzw. nicht mehr einnehmen kann.

Die Stimulationsenergie wird in diesen Fällen zunächst in das Implantat eingekoppelt und dann in der entsprechenden Form aus diesem ausgekoppelt und direkt an den zu behandelnden Bereich abgegeben. Dies hat den Vorteil, dass sich die Wirkung der Stimulation gerade auf die zu behandelnde Umgebung des Implantats konzentriert, während andere Bereiche des Körpers nicht betroffen sind.

Die Einkopplung der Stimulationsenergie in das Implantat, also beispielsweise den Stent, kann auf verschiedene Weise erfolgen. So ist bei bevorzugten Varianten der erfindungsgemäßen Vorrichtung vorgesehen, dass die Erregereinrichtung eine Induktionseinrichtung zur induktiven Einkopplung der Stimulationsenergie in das Implantat umfasst. Mit anderen Worten werden durch entsprechende magnetische Wechselfelder entsprechende Ströme in dem geeignet ausgestalteten Implantat induziert, die wiederum direkt oder über entsprechende aktive und/oder passive Elemente des Implantats zur Erzeugung der entsprechenden Stimulationsströme verwendet werden. Hierbei kann schon das magnetische Wechselfeld entsprechend niedrigfrequent und/oder niedrigfrequent moduliert sein. Zusätzlich oder alternativ können auch mehrere elektrische Felder in dem Implantat induziert werden, die sich dann zu einem entsprechend niedrigfrequent modulierten elektrischen Wechselfeld überlagern. Dies kann beispielsweise erfolgen, indem wenigstens zwei hochfrequente elektrische Wechselfelder mit entsprechend geringem Frequenzunterschied erzeugt und einander nach Art einer Schwebung überlagert werden.

Bei anderen bevorzugten Varianten der erfindungsgemäßen Vorrichtung umfasst die Erregereinrichtung eine Sendeeinrichtung zur Einkopplung der Stimulationsenergie in das Implantat in Form elektromagnetischer Schwingungen. Das Implantat umfasst dabei ein nach Art einer Antenne ausgebildetes Antennenelement. Mit anderen Worten werden durch elektromagnetische Schwingungen entsprechende Ströme in dem Antennenelement des Implantats erzeugt, die wiederum gegebenenfalls über geeignete aktive und/oder passive Elemente des Implantats zur Erzeugung der gewünschten Stimulationsströme verwendet werden. Hierbei kann schon die elektromagnetische Erregerschwingung entsprechend niedrigfrequent moduliert sein. Zusätzlich oder alternativ können auch hier wiederum mehrere elektrische Ströme in dem Implantat erzeugt werden, die einander dann zu einem entsprechend niedrigfrequenten elektrischen Wechselstrom überlagert werden. Dies kann beispielsweise erfolgen, indem wenigstens zwei hochfrequente elektrische Wechselströme mit entsprechend geringem Frequenzunterschied erzeugt und einander nach Art einer Schwebung überlagert werden.

Die Sendefrequenz der Sendeeinrichtung ist vorzugsweise so gewählt, dass die Frequenz der von dem betreffenden Antennenelement empfangenen elektromagnetischen Wellen der Resonanzfrequenz des Antennenelements entspricht, da hiermit eine optimale Energieeinkopplung erzielt werden kann. Es versteht sich jedoch, dass auch mit einer gewissen Fehlanpassung zwischen Sendefrequenz und Resonanzfrequenz des Antennenelements gearbeitet werden kann. Hierbei ist im Übrigen zu beachten, dass die Frequenz der von dem Antennenelement empfangenen elektromagnetischen Wellen aufgrund der Veränderung durch das Dielektrikum des Körpergewebes nicht mit der Sendefrequenz (im Vakuum) der Sendeeinrichtung übereinstimmt.

Bevorzugt ist eine Einrichtung zum Fokussieren der elektromagnetischen Schwingungen im Bereich des Implantats vorgesehen, um den Energiefluss durch nicht im Behandlungsbereich gelegenes Gewebe im Hinblick auf eine möglichst geringe Beeinträchtigung des im Übertragungsweg liegenden nicht zu behandelnden Gewebes zu reduzieren. Diese Einrichtung zum Fokussieren elektromagnetischer Schwingungen kann in beliebiger bekannter Weise ausgebildet sein. Bevorzugt handelt es sich um einen elliptischen Reflektor, in dessen Brennpunkten die Sendeeinrichtung und das Implantat angeordnet sind.

Vorzugsweise ist die Erregereinrichtung zur Bewirkung von Stimulationsströmen ausgebildet, deren Frequenz und/oder Modulationsfrequenz im Bereich der Frequenzen liegt, bei denen die Ausschüttung von die Zellproliferation steuernden sekundären Botenstoffen in den glatten Muskelzellen und zusätzlich oder alternativ in den Endothelzellen sowie zusätzlich oder alternativ in den Fibroblasten des Gefäßes beeinflusst wird, da Wucherungen dieser Zelltypen beispielsweise den Hauptanteil an der Stenosenbildung, insbesondere in Blutgefäßen haben. Alternativ kann das Zellwachstum durch entsprechende Wahl der Frequenzen auch angeregt werden, beispielsweise um die Wundheilung zu beschleunigen. Ebenso kann es gewünscht sein, geschwächte Gefäßabschnitte, wie sie beispielsweise häufig bei der Bildung von Aneurysmen eine Rolle spielen, durch erhöhtes Zellwachstum zu verstärken, um ihre Widerstandsfähigkeit gegenüber den auf die Gefäßwand einwirkenden Belastungen zu erhöhen.

Bevorzugt ist die Erregereinrichtung dabei zur Bewirkung von Stimulationsströmen in dem zu behandelnden Bereich des Gefäßes mit einer Frequenz und/oder Modulationsfrequenz bis zu 200 Hz, vorzugsweise zwischen 10 und 100 Hz, ausgebildet, da in diesem Bereich hinsichtlich der Ausschüttung bzw. Hemmung der genannten sekundären Botenstoffe "Resonanzfrequenzen" der Zellen liegen, bei denen besonders gute Ergebnisse erzielt werden können. Die Erfindung kann zur Behandlung unterschiedlichster Gefäße des menschlichen oder tierischen Körpers eingesetzt werden, besonders vorteilhaft lässt sie sich aber in Verbindung mit der Behandlung von Blutgefäßen einsetzen.

Bei bevorzugten Varianten der erfindungsgemäßen Vorrichtung weist die Erregereinrichtung eine Zeitsteuereinrichtung auf, die zur schrittweisen oder kontinuierlichen Reduktion der Stimulationsintensität und zusätzlich oder alternativ zur schrittweisen oder kontinuierlichen Reduktion der Stimulationshäufigkeit ausgebildet ist. Hiermit ist ein so genanntes "Ausschleichen" der Behandlung möglich, bei dem die künstliche Unterdrückung bzw. Anregung von Zellproliferationsprozessen schrittweise oder kontinuierlich verringert wird, um ein überschießendes Wachstum der Zellen bzw. ein völliges Aussetzen des Zellwachstums als Antwort auf ein abruptes Absetzen der Behandlung zu verhindern.

Bevorzugt wird die erfindungsgemäße Vorrichtung mit einer Stimulationseinrichtung zur direkten Induktion der Stimulationsströme in dem Körpergewebe im Zusammenhang mit einem erfindungsgemäßen Implantat zum Einsetzen in das betreffende Gefäß verwendet. Hierbei kann es sich beispielsweise um einen Stent handeln. Das Implantat weist einen zum Anliegen an der Wandung des Gefäßes vorgesehenen rohrförmigen Körper auf. Der rohrförmige Körper besteht zumindest abschnittsweise aus einem weichmagnetischen Material. Dieses bewirkt wiederum eine Konzentration des Magnetfeldes im rohrförmigen Körper des Implantats und in dessen Umgebung, so dass insbesondere im Außenraum des Implantats in unmittelbarer Nähe der Implantatoberfläche ein größeres elektrisches Feld induziert wird als bei einem Gefäß ohne ein solches Implantat. Hierdurch ergibt sich gerade in dem Bereich, der stimuliert werden soll, eine vorteilhafte Verstärkung der Stimulationsströme.

Die Erfindung betrifft weiterhin ein Implantat zum Einsetzen in ein Gefäß des menschlichen oder tierischen Körpers. Bei dem Gefäß handelt es sich um ein Blutgefäß. Das Implantat ist ein Stent, der noch weitere Funktionen erfüllt bzw. erfüllen kann.

Die oben genannte Aufgabe wird mit dem erfindungsgemäßen Implantat dadurch gelöst, dass das Implantat zum Zellproliferationsmechanismen beeinflussenden Stimulieren von Zellen des Gefäßes, in das es implantiert ist, mittels Stimulationsströmen ausgebildet ist die von einer Energieeinrichtung nach Anspruch 1 bewirkt werden. Die Stimulation kann dabei in der oben beschriebenen Weise sowohl zu Hemmung als auch zur Anregung der Zellproliferationsmechanismen dienen. Besonders vorteilhaft ist diese Gestaltung wie schon erwähnt dann, wenn das Implantat ein Stent ist, der ohnehin schon aus anderen Gründen im zu behandelnden Bereich des Gefäßes implantiert werden muss. Dies ist z. B. dann der Fall, wenn der Stent erforderlich ist, um das Gefäß gegen eine Rückstellkraft aufgeweitet zu halten.

Das Implantat kann hierzu mit einer eigenen entsprechend langlebigen oder wiederaufladbaren Energieversorgung und einer geeigneten Steuerschaltung zur Steuerung der Energieabgabe versehen sein.

Bei anderen bevorzugten, weil klein bauenden und einfach zu realisierenden Varianten ist das Implantat zum Auskoppeln induktiv eingekoppelter Stimulationsenergie in Form von, vorzugsweise niedrigfrequenten, Stimulationsströmen ausgebildet. Dabei werden durch extern erzeugte magnetische Wechselfelder Wechselströme in dem Implantat induziert. Hierzu kann beispielsweise ein entsprechend niedrigfrequent moduliertes magnetisches Wechselfeld vorgesehen sein. Die induzierten Wechselströme werden wiederum direkt oder über entsprechende aktive und/oder passive Elemente des Implantats zur Erzeugung der entsprechenden Stimulationsströme verwendet. Handelt es sich bei dem Implantat beispielsweise um einen Stent, können diese Elemente in einer entsprechenden Halbleiterbeschichtung oder Halbleiterschicht des herkömmlich gestalteten Stentkörpers eingebettet sein. Ebenso können sie auf einem gesonderten, beim Implantieren des Stents vorzugsweise mechanisch nicht oder nur wenig belasteten Trägerelement am Stent angeordnet sein.

Zusätzlich oder alternativ können auch mehrere Wechselströme in dem Implantat induziert werden, die dann zu einem entsprechend niedrigfrequent modulierten elektrischen Wechselstrom überlagert werden. Dies kann beispielsweise erfolgen, indem wenigstens zwei hochfrequente Wechselströme mit entsprechend geringem Frequenzunterschied erzeugt und einander nach Art einer Schwebung überlagert werden.

Das Implantat kann mit wenigstens einem induktiven Element versehen sein, das mit einem kapazitiven Element einen Resonanzkreis bildet. Zur Erzeugung niedrigfrequent modulierter Stimulationsströme kann die Resonanzfrequenz des Resonanzkreises im Bereich einer hochfrequenten Trägerfrequenz liegen, welche dann durch eine entsprechende Modulation der Anregungsamplitude entsprechend niedrigfrequent moduliert wird. Zur beschriebenen Überlagerung der Ströme können auch mehrere solcher Resonanzkreise vorgesehen sein, die dann zur Überlagerung miteinander verschaltet sind. Handelt es sich bei dem Implantat beispielsweise um einen Stent, können diese induktiven und kapazitiven Elemente in einer entsprechenden Beschichtung oder Schicht des Implantats eingebettet sein. Ebenso können sie auf einem gesonderten, beim Implantieren des Stents vorzugsweise mechanisch nicht oder nur wenig belasteten Trägerelement am Stent angeordnet sein.

Bevorzugt umfasst das erfindungsgemäße Implantat einen zum Anliegen an der Wandung des Gefäßes vorgesehenen rohrförmigen Körper, der zur Ausbildung eines Resonanzkreises zumindest abschnittsweise als Induktionsspule ausgebildet ist. Hierdurch steht ein relativ großer Raum für die Ausbildung der Induktivität zur Verfügung, was sich dank höherer möglicher Windungszahlen sowie Spulen- und Leiterquerschnitte positiv auf den maximalen Energieeintrag in die Spule auswirkt. Hierbei kann das Implantat selbst zur Ausbildung der Spule als Helix ausgebildet sein. Es ist aber auch möglich, die Wicklung durch eine Beschichtung mit entsprechenden leitenden Windungsabschnitten auf dem dann beliebig gestalteten rohrförmigen Körper auszubilden. Ebenso ist es möglich, die Wicklung durch entsprechend leitende Abschnitte des dann beliebig gestalteten rohrförmigen Körpers auszubilden.

Die Enden der betreffenden Induktionsspule können zur Ausbildung des Resonanzkreises mit einem an beliebiger Stelle des Implantats angeordneten kapazitiven Element verschaltet sein. Es ist jedoch auch möglich, anstelle eines diskreten Kondensators an den Wicklungsenden der Spule jeweils nur kleine Plättchen oder Pads auszubilden. Deren Grenzschichtkapazitäten bilden dann zusammen mit der elektrischen Verbindung durch das Körpergewebe bzw. die Körperflüssigkeit im Gefäßzwei hintereinander geschaltete Kondensatoren. Zur Erhöhung der Kapazität können die Pads mit einer fraktalen Oberfläche versehen sein, wie sie beispielsweise für Elektroden von Herzschrittmachern verwendet wird. Zur Erhöhung der Spannungsfestigkeit können die Pads zusätzlich mit einer dünnen Isolierschicht versehen sein.

Andere vorteilhafte Varianten des erfindungsgemäßen Implantats zeichnen sich dadurch aus, dass das Implantat zum Auskoppeln mittels hochfrequenter elektromagnetischer Wellen eingekoppelter Stimulationsenergie in Form niedrigfrequenter und/oder niedrigfrequent modulierter Stimulationsströme ausgebildet ist, wobei es zum Einkoppeln der Stimulationsenergie ein nach Art einer Antenne ausgebildetes Antennenelement umfasst.

Hierbei wird eine externe Sendeeinrichtung zur Einkopplung der Stimulationsenergie in Form elektromagnetischer Schwingungen in das demgemäß zumindest abschnittsweise nach Art einer Antenne ausgebildete Implantat verwendet. Mit anderen Worten werden durch entsprechende elektromagnetische Schwingungen Ströme in dem Antennenelement erzeugt. Hierbei kann die elektromagnetische Erregerschwingung schon entsprechend niedrigfrequent moduliert sein. Die so erzeugten Ströme werden wiederum direkt oder über entsprechende aktive und/oder passive Elemente des Implantats zur Erzeugung der entsprechenden Stimulationsströme verwendet. Handelt es sich bei dem Implantat beispielsweise um einen Stent, können diese aktiven und/oder passiven Elemente in einer entsprechenden Halbleiterbeschichtung oder Halbleiterschicht des herkömmlich gestalteten Stentkörpers eingebettet sein. Ebenso können sie auf einem gesonderten, beim Implantieren des Stents vorzugsweise mechanisch nicht oder nur wenig belasteten Trägerelement am Stent angeordnet sein.

Zusätzlich oder alternativ können durch elektromagnetische Schwingungen unterschiedlicher Frequenz auch mehrere Wechselströme in dem Implantat erzeugt werden, die dann zu einem entsprechend niedrigfrequent modulierten elektrischen Wechselstrom überlagert werden. Dies kann beispielsweise erfolgen, indem wenigstens zwei hochfrequente Wechselströme mit entsprechend geringem Frequenzunterschied erzeugt und einander nach Art einer Schwebung überlagert werden.

Das Implantat kann mit wenigstens einem Antennenelement versehen sein. Zur beschriebenen Überlagerung der Ströme können auch zwei solcher Antennenelemente vorgesehen sein, die dann zur Überlagerung miteinander verschaltet sind.

Handelt es sich bei dem Implantat beispielsweise um einen Stent, können diese Antennenelemente in einer entsprechenden Beschichtung oder Schicht des Implantats eingebettet sein. Ebenso können sie auf einem gesonderten, beim Implantieren des Stents vorzugsweise mechanisch nicht oder nur wenig belasteten Trägerelement am Stent angeordnet sein.

In einer bevorzugten, weil einfach herzustellenden Konfiguration umfasst das Implantat einen zum Anliegen an der Wandung des Gefäßes vorgesehenen rohrförmigen Körper, der zumindest abschnittsweise nach Art einer Dipolantenne ausgebildet ist. Hierbei kann der gesamte Körper als Antenne fungieren, wobei dann in der Mitte eine entsprechend isolierende Verbindung zwischen den beiden Körperhälften vorgesehen sein muss. Es ist jedoch auch möglich, eine durch eine isolierende Schicht vom rohrförmigen Grundkörper getrennte leitende Schicht oder eine auf einem nicht leitenden rohrförmigen Grundkörper angeordnete leitende Schicht nach Art einer Dipolantenne auszubilden.

Bei bevorzugten Weiterbildungen des erfindungsgemäßen Implantats ist eine Auskopplungseinheit vorgesehen, die eine Umwandlungseinheit zum Umwandeln der eingekoppelten Stimulationsenergie in niedrigfrequente und/oder niedrigfrequent modulierte Stimulationsströme umfasst. Bei der Umwandlungseinheit kann es sich um die oben beschriebenen aktiven oder passiven Elemente handeln. Die Umwandlungseinheit umfasst dabei bevorzugt eine elektronische Schaltung zum Umwandeln eines hochfrequenten Stromes in einen niedrigfrequenten und/oder niedrigfrequent modulierten Stimulationsstrom. Diese ist weiter vorzugsweise in einer Beschichtung des Implantats ausgebildet.

Es kann sich bei der Umwandlungseinheit sich jedoch auch einfach um eine oben ebenfalls beschriebene Verschaltung zur Überlagerung zweier Wechselströme handeln.

Hinsichtlich der Frequenzen bzw. Modulationsfrequenzen der durch das Implantat auszukoppelnden Stimulationsströme ist anzumerken, dass diese in den oben bereits zur erfindungsgemäßen Vorrichtung beschriebenen Bereichen liegen.

Die vorliegende Erfindung betrifft weiterhin eine Anordnung mit einer erfindungsgemäßen Stimulationsvorrichtung und einem dieser Stimulationsvorrichtung angepassten erfindungsgemäßen Implantat.

Weitere bevorzugte Ausgestaltungen der vorliegenden Erfindung ergeben sich aus den Unteransprüchen und der nachstehenden Beschreibung bevorzugter Varianten der Erfindung unter Bezugnahme auf die beigefügten Zeichnungen. Es zeigen:
- Figur 1: einen schematischen Teilschnitt durch eine Anordnung aus einer erfindungsgemäßen Stimulationsvorrichtung und einem erfindungsgemäßen Implantat;
- Figur 2: eine schematische Ansicht einer bevorzugten Ausführungsform des Implantats aus Figur 1;
- Figur 3: eine schematische Ansicht eines Details einer weiteren bevorzugten Ausführungsform eines erfindungsgemäßen Implantats;
- Figur 4: eine schematische Ansicht einer bevorzugten Ausführungsform eines erfindungsgemäßen Implantats;
- Figur 5: einen schematischen Teilschnitt durch eine weitere Anordnung aus einer erfindungsgemäßen Stimulationsvorrichtung und einem erfindungsgemäßen Implantat;
- Figur 6: eine schematische Ansicht einer bevorzugten Ausführungsform des Implantats aus Figur 5;
- Figur 7: eine schematische Ansicht einer weiteren bevorzugten Ausführungsform eines erfindungsgemäßen Implantats.

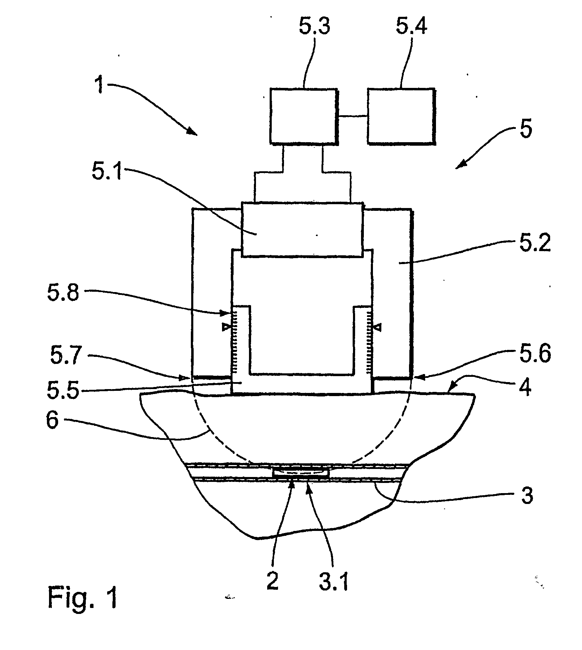

Figur 1 zeigt einen schematischen Teilschnitt durch eine Anordnung aus einer erfindungsgemäßen Vorrichtung 1 zum Verhindern oder Verlangsamen der Ausbildung von Stenosen und einem erfindungsgemäßen Implantat in Form eines Stents 2, der in einem zu behandelnden Blutgefäß 3 in einer gewissen Tiefe unter der Körperoberfläche 4 eines Patienten angeordnet ist.

Die Vorrichtung 1 umfasst eine Erregereinrichtung 5, die zur berührungslosen Bewirkung von Stimulationsströmen in einem zu behandelnden Bereich 3.1 des Gefäßes 3 ausgebildet ist. Hierzu umfasst sie einen Elektromagneten aus einer Spule 5.1 und einem hufeisenförmigen Kern 5.2 sowie eine mit der Spule 5.1 verbundene Versorgungseinrichtung 5.3, die über eine Steuereinrichtung 5.4 gesteuert wird.

Die Erregereinrichtung 5 umfasst weiterhin eine Positioniereinrichtung 5.5, welche auf die Körperoberfläche 4 des Patienten aufgesetzt werden kann und über welche die Pole 5.6 und 5.7 des Elektromagneten bezüglich des Behandlungsbereichs 3.1 positioniert werden können. Um das Positionieren zu erleichtern, sind Skalen 5.8 vorgesehen.

Im gezeigten Beispiel ist die Erregereinrichtung 5 so ausgebildet, dass sie ein Magnetfeld mit einer Trägerfrequenz oberhalb von 1 kHz und geeigneter niedrigfrequenter Modulation erzeugt, welches an den Polen 5.6 und 5.7 des Elektromagneten austritt und etwa einen Raum ausfüllt, wie er durch die Kontur 6 angedeutet ist. Der Durchmesser des durch die Kontur 6 angedeuteten Raumes entspricht etwa dem Abstand der beiden Pole 5.6 und 5.7. Die Pole 5.6 und 5.7 sind über die Positioniereinrichtung 5.5 so bezüglich des Behandlungsbereichs 3.1 positioniert, dass die Kontur 6 im Bereich des Stents 2 verläuft.

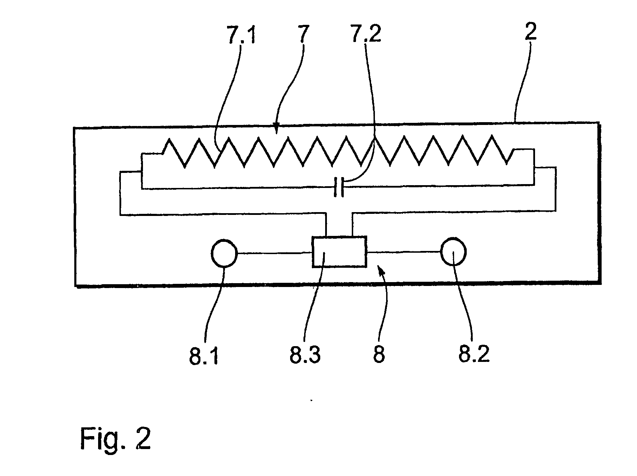

Der Stent 2 ist zum Auskoppeln der über das Magnetfeld induktiv eingekoppelten, von der Erregereinrichtung 5 abgegebenen Stimulationsenergie in Form niedrigfrequent modulierter Stimulationsströme ausgebildet. Zur Einkopplung der Stimulationsenergie ist er, wie Figur 2 in schematischer Weise zu entnehmen ist, mit einem Resonanzkreis 7 versehen, der aus einem induktiven Element 7.1 und einem kapazitiven Element 7.2 besteht und dessen Resonanzfrequenz der Trägerfrequenz des Magnetfeldes entspricht. Dieser Resonanzkreis 7 ist mit einer Auskopplungseinheit 8 verbunden. Diese umfasst Elektroden 8.1 und 8.2 sowie eine Umwandlungseinheit 8.3, welche die im Resonanzkreis 7 induzierten hochfrequenten Ströme gegebenenfalls in niedrigfrequente Stimulationsströme umwandelt, die dann über die Elektroden 8.1 und 8.2 an das Blutgefäß 3 abgegeben werden.

Die Umwandlungseinheit 8.3 umfasst eine elektronische Schaltung, welche die im Resonanzkreis 7 induzierten hochfrequenten Ströme gegebenenfalls in niedrigfrequente Stimulationsströme umwandelt. Hierzu umfasst die elektronische Schaltung an sich bekannte passive Schaltungselemente. Es versteht sich jedoch, dass bei anderen Varianten auch aktive Schaltungselemente oder eine Kombination aus aktiven und passiven Schaltungselementen verwendet werden können.

Der Resonanzkreis 7 und die Auskopplungseinheit 8 sind in entsprechenden Beschichtungen auf dem Körper des Stents 2 ausgebildet. Es versteht sich jedoch, dass bei anderen Varianten beispielsweise der Stentkörper selbst oder ein Abschnitt des Stentkörpers das induktive und/oder das kapazitive Element darstellen kann.

Weiterhin versteht es sich, dass bei anderen Varianten des erfindungsgemäßen Stents auch mehrere Resonanzkreise vorgesehen sein können. Diese können dann beispielsweise so ausgebildet sein, dass sie nur leicht unterschiedliche Resonanzfrequenzen aufweisen. Diese Resonanzkreise werden dann lediglich entsprechend verschaltet, so dass sich ihre induzierten Wechselströme einander nach Art einer Schwebung mit der entsprechenden Modulationsfrequenz überlagern.

Die Modulationsfrequenz der Stimulationsströme liegt im Bereich der Frequenzen, bei denen die Ausschüttung von die Zellproliferation steuernden sekundären Botenstoffen, wie dem zyklischem Adenosinmonophosphat (cAMP), in den Zellen des Gefäßes beeinflusst wird. Im gezeigten Beispiel ist nur eine Auskopplungseinheit dargestellt. Es versteht sich, dass bei anderen Varianten auch mehrere solcher Auskopplungseinheiten vorgesehen sein können, da die zur Beeinflussung erforderliche Frequenz von Zelltyp zu Zelltyp abweichen kann, und gegebenenfalls mehrere Zelltypen entsprechend stimuliert werden müssen.

Für Blutgefäße, wie das Blutgefäß 3, liegt die Frequenz bzw. Modulationsfrequenz der Stimulationsströme bevorzugt im Bereich der Frequenzen, bei denen die Ausschüttung von die Zellproliferation steuernden sekundären Botenstoffen in den glatten Muskelzellen und zusätzlich oder alternativ in den Endothelzellen sowie zusätzlich oder alternativ in den Fibroblasten des Gefäßes angeregt wird, da Wucherungen dieser Zelltypen den Hauptanteil an der Stenosenbildung, insbesondere in Blutgefäßen haben.

Es versteht sich, dass bei anderen Varianten die Ausschüttung dieser sekundären Botenstoffe auch gehemmt werden kann, um wie eingangs beschrieben eine Anregung des Zellwachstums zu erzielen. Ebenso versteht es sich, dass die Konzentration anderer Botenstoffe auch einen gegenläufigen Einfluss auf das Zellwachstum bzw. die Zellteilungsaktivität der Zellen haben können und demgemäß die Ausschüttung dieser Botenstoffe in der entsprechenden gegenläufigen Weise beeinflusst wird.

Die Intensität des durch die Erregereinrichtung 5 erzeugten Magnetfeldes ist im gezeigten Beispiel so gewählt, dass die Stimulationsströme im zu stimulierenden Gewebe eine Stromdichte von wenigstens 5 µA/cm² erreichen.

Die Steuereinrichtung 5.4 kann die Stimulation mit beliebigen vorgebbaren zeitlichen Stimulationsabläufen steuern. Sie weist insbesondere eine nicht dargestellte Zeitssteuerschaltung auf, die nach einer bestimmten vorgebbaren Stimulationsdauer zur schrittweisen Reduktion der Stimulationsintensität ausgebildet ist. Hiermit ist ein so genanntes "Ausschleichen" der Behandlung möglich, bei dem die künstliche Unterdrückung von Zellproliferationsprozessen schrittweise verringert wird, um ein überschießendes Wachstum der Zellen als Antwort auf ein abruptes Absetzen der Behandlung zu verhindern. Es versteht sich, dass sie bei anderen Varianten auch zur kontinuierlichen Reduktion der Stimulationsintensität und zusätzlich oder alternativ auch zur schrittweisen oder kontinuierlichen Reduktion der Stimulationshäufigkeit ausgebildet sein kann.

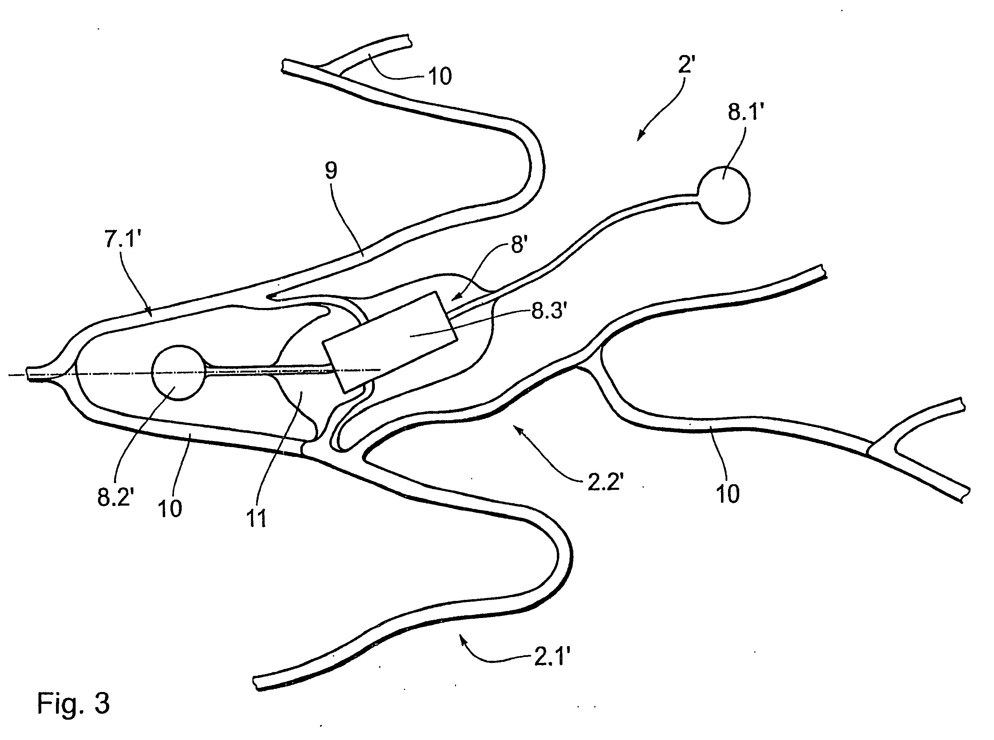

Figur 3 zeigt ein Detail einer Abwicklung der Mantelfläche eines erfindungsgemäßen Stents 2', der in bekannter Weise aus mäanderförmig in Umfangsrichtung des Stents 2' verlaufenden Stegelementen 2.1' besteht, die in Längsrichtung des Stents 2' durch Verbindungsstege 2.2' miteinander verbunden sind. Der Stent 2' ist dabei in der oben zu Figur 1 und 2 beschriebenen Weise zur induktiven Einkopplung von Stimulationsenergie ausgebildet, so dass hier lediglich auf seine Besonderheiten eingegangen werden soll.

Das induktive Element 7.1' ist von einer leitenden Schicht 9 auf den Stegelementen 2.1' und den Verbindungsstegen 2.2' gebildet, wobei zur Ausbildung einer Spule nicht leitende Bereiche 10 auf den Stegelementen 2.1' und den Verbindungsstegen 2.2' vorgesehen sind. Die Auskopplungseinheit 8' ist mit der leitenden Schicht 9 verbunden, wobei sie auf einem mechanisch kaum belasteten Pad 11 angeordnet, welches aus dem Material der Stegelemente besteht und an diesen angeschlossen ist. Die Stimulationselektroden 8.1' und 8.2' sind mit der Umwandlungseinheit 8.3' der Auskopplungseinheit 8' verbunden und durch eine leitende Schicht auf entsprechenden Fortsätzen des Pads 11 ausgebildet.

Die elektronischen Schaltungselemente der Umwandlungseinheit 8.3' sind dabei in einer entsprechenden halbleitenden SiC-Beschichtung des Pads ausgebildet. Das Material der Stegelemente 2.1' bzw. der Verbindungsstege 2.2' kann dabei elektrisch isolierend sein. Es versteht sich jedoch, dass bei anderen Varianten auch einfach eine isolierende Schicht zwischen dem Grundmaterial und der leitenden Schicht 9 vorgesehen sein kann. Weiterhin versteht es sich, dass der gesamte Stent zumindest auf seiner dem Gefäß zugewandten Seite bis auf die Stimulationselektroden mit einer zusätzlichen isolierenden Beschichtung versehen sein kann.

Es versteht sich, dass über den Stent mehrere solcher Auskopplungseinheiten mit Stimulationselektroden verteilt sein können, um eine großflächige Stimulation zu erzielen.

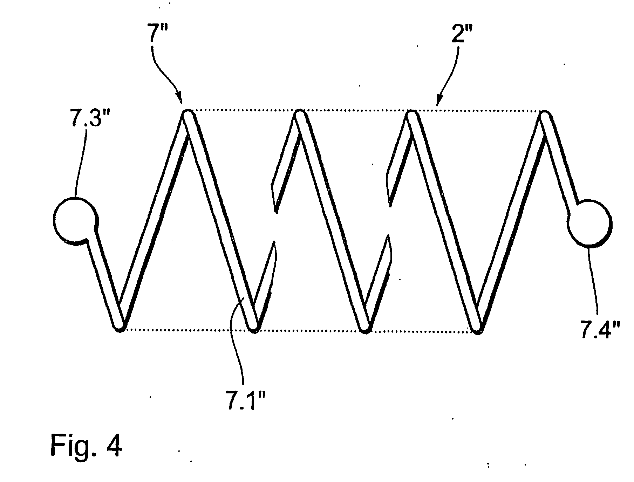

Figur 4 zeigt eine weitere bevorzugte Ausführung eines erfindungsgemäßen Stents 2" zur induktiven Einkopplung der Stimulationsenergie. In ihrer grundsätzlichen Funktionsweise unterscheidet sich diese Variante nicht von den zu den Figuren 1 bis 3 beschriebenen Varianten, so dass auch hier lediglich auf die Unterschiede bzw. Besonderheiten eingegangen werden soll.

Der Körper des Stents 2" ist hier nach Art einer Wendel aus einem elektrisch leitfähigen Material ausgebildet. Er bildet somit das induktive Element 7.1" für den Resonanzkreis 7". Das kapazitive Element des Resonanzkreises 7" ist von zwei Pads 7.3" und 7.4" am jeweiligen Ende des Stents 2" gebildet. Deren Grenzschichtkapazitäten bilden zusammen mit der elektrischen Verbindung durch das Körpergewebe bzw. die Körperflüssigkeit im Gefäß zwei hintereinander geschaltete Kondensatoren. Zur Erhöhung der Kapazität sind die Pads 7.3" und 7.4" mit einer fraktalen Oberfläche versehen sein, wie sie beispielsweise für Elektroden von Herzschrittmachern verwendet wird.

Auch bei dieser Variante kann eine in der oben beschriebenen Weise gestaltete Auskopplungseinheit vorgesehen sein. Es versteht sich jedoch, dass diese bei entsprechend gewählter Frequenz bzw. Modulationsfrequenz des Magnetfeldes auch entfallen kann und dann die Pads auch die Stimulationselektroden bilden.

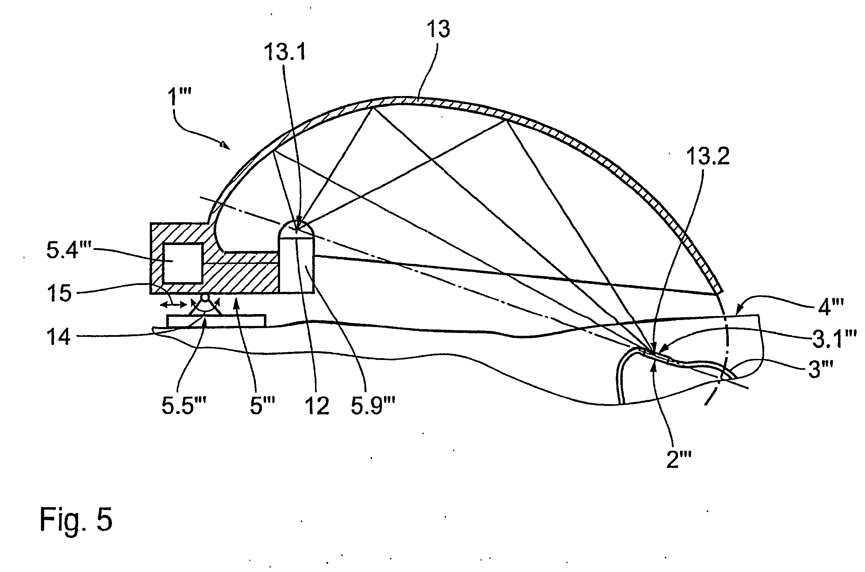

Figur 5 zeigt einen schematischen Teilschnitt durch eine Anordnung aus einer erfindungsgemäßen Vorrichtung 1''' zum Verhindern oder Verlangsamen der Ausbildung von Stenosen und einem erfindungsgemäßen Implantat in Form eines Stents 2''', der in einem zu behandelnden Blutgefäß 3''' in einer gewissen Tiefe unter der Körperoberfläche 4''' eines Patienten angeordnet ist. Der Stent 2''' umfasst ein - nicht dargestelltes - nach Art einer Antenne ausgebildetes Antennenelement, das zur Einkopplung von Stimulationsenergie in Form elektromagnetischer Wellen ausgebildet ist.

Die Vorrichtung 1''' weist eine Erregereinrichtung 5''' auf, die zur berührungslosen Bewirkung niedrigfrequenter Stimulationsströme in einem zu behandelnden Bereich 3.1''' des Gefäßes 3''' ausgebildet ist. Hierzu umfasst sie einen Sender 5.9''', der in den Halbraum oberhalb der Kontur 12 elektromagnetische Wellen aussenden kann, eine mit dem Sender 5.9''' verbundene Steuereinrichtung 5.4''' sowie einen dem Sender 5.9''' zugeordneten Reflektor 13. Dieser Reflektor 13 ist von einem Teil eines Rotationsellipsoids gebildet, wobei der Sender 5.9''' in dem ersten Brennpunkt 13.1 dieses Rotationsellipsoids angeordnet ist. Die Erregereinrichtung 5''' ist so angeordnet, dass der Stent 2''' in dem zweiten Brennpunkt 13.2 des Rotationsellipsoids angeordnet ist. Hierdurch sind die elektromagnetischen Wellen des Senders 5.9''' am Ort des Stents 2''' fokussiert.

Es versteht sich, dass der Reflektor bei anderen Varianten eine vom Rotationsellipsoid abweichende Form haben kann. Diese ist vorzugsweise so gewählt, dass die durch die unterschiedlichen Wellenlängen in Luft und Körper verursachte Defokussierung ausgeglichen wird.

Die Erregereinrichtung 5''' umfasst weiterhin eine Positioniereinrichtung 5.5''', welche auf die Körperoberfläche 4''' des Patienten aufgesetzt werden kann. Über diese Positioniereinrichtung 5.5''' kann zur Anpassung an unterschiedliche Positionen des Stents 2''' bzw. des Behandlungsbereichs 3.1''' durch Verschwenken in Richtung des Pfeiles 14 bzw. Verschieben in Richtung des Pfeiles 15 die Lage des zweiten Brennpunktes 13.2 des Reflektors verändert werden. Zusätzlich lässt sich durch Drehen des Senders 5.9''' in der Ebene der Kontur 12 die gegenseitige Polarisation von Sender zu Empfänger optimal ausrichten.

Im gezeigten Beispiel ist die Erregereinrichtung 5''' so ausgebildet, dass sie Stimulationsenergie in Form hochfrequenter elektromagnetischer Wellen abgibt, welche auf den Stent 2''' fokussiert sind und in diesen eingekoppelt werden. Der Stent 2''' wiederum ist zum Auskoppeln der eingekoppelten, von der Erregereinrichtung 5''' abgegebenen Stimulationsenergie in Form niedrigfrequenter Stimulationsströme ausgebildet.

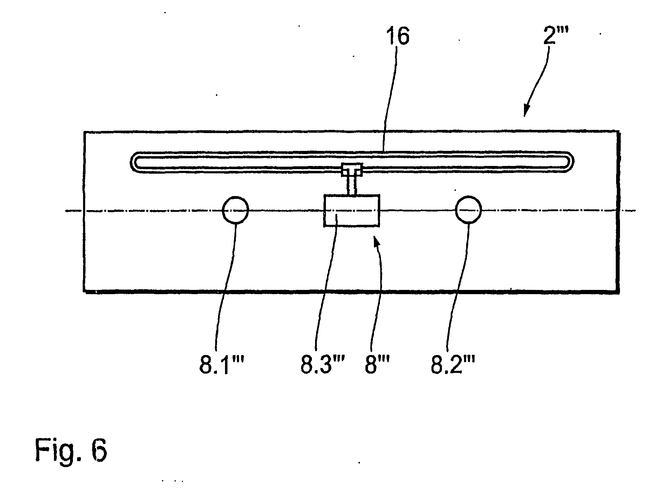

Zur Einkopplung der Stimulationsenergie ist der Stent 2''', wie Figur 6 in schematischer Weise zu entnehmen ist, mit einem Faltdipol 16 versehen. Dessen Länge entspricht einem Viertel der Wellenlänge der an dem Faltdipol 16 auftreffenden, von dem Sender 5''' erzeugten elektromagnetischen Wellen. Hierbei entspricht die von dem Faltdipol 16 empfangene Frequenz aufgrund der Veränderung durch das Dielektrikum des Körpergewebes nicht der Sendefrequenz (im Vakuum) des Senders 5'''. Der Faltdipol 16 ist mit einer Auskopplungseinheit 8''' verbunden, welche die im Faltdipol 16 erzeugten hochfrequenten Ströme in niedrigfrequente Stimulationsströme umwandelt, die dann über die Elektroden 8.1''' und 8.2''' an das Blutgefäß 3''' abgegeben werden.

Die Umwandlungseinheit 8.3''' der Auskopplungseinheit 8''' umfasst eine elektronische Schaltung, welche die im Faltdipol 16 erzeugten hochfrequenten Ströme in niedrigfrequente Stimulationsströme umwandelt. Hierzu umfasst die elektronische Schaltung geeignete, an sich bekannte passive Schaltungselemente. Es versteht sich jedoch, dass bei anderen Varianten auch aktive Schaltungselemente oder eine Kombination aus aktiven und passiven Schaltungselementen verwendet werden können.

Der Faltdipol 16 und die Auskopplungseinheit 8''' sind in entsprechenden Beschichtungen bzw. Schichten auf dem Grundkörper des Stents 2''' ausgebildet. So besteht der Faltdipol 16 aus einer entsprechend geformten leitenden Beschichtung auf dem Grundkörper des Stents 2'''. Hierbei besteht auch der Grundkörper des Stents 2''' aus einem elektrisch leitenden Material, der Faltdipol 16 ist hiervon jedoch durch eine isolierende Zwischenlage getrennt. Bei anderen Varianten kann der Grundkörper des Stents selbst schon aus einem entsprechend isolierenden Material bestehen. Es versteht sich im Übrigen, dass bei anderen Varianten beispielsweise der Stentkörper selbst oder ein Abschnitt des Stentkörpers den Dipol darstellen kann.

Weiterhin versteht es sich, dass bei anderen Varianten des erfindungsgemäßen Stents auch mehrere Dipole vorgesehen sein können. Diese können dann beispielsweise so ausgebildet sein, dass sie nur leicht unterschiedliche Resonanzfrequenzen aufweisen. Diese Dipole werden dann lediglich entsprechend verschaltet, so dass sich die in ihnen erzeugten Wechselströme einander nach Art einer Schwebung zu einem Stimulationsstrom mit der gewünschten Stimulationsfrequenz überlagern. Es versteht sich hierbei wiederum, dass dann auch der Sender entsprechend ausgebildet sein muss, um elektromagnetische Wellen mit zwei hierzu geeigneten Frequenzen auszusenden.

Weiterhin versteht es sich, dass bei weiteren Varianten ist die Erregereinrichtung so ausgebildet sein kann, dass sie Stimulationsenergie in Form niedrigfrequent modulierter hochfrequenter elektromagnetischer Wellen abgibt, welche auf den Stent fokussiert sind und in diesen eingekoppelt werden. Der Stent wiederum ist dann zum Auskoppeln der eingekoppelten, von der Erregereinrichtung abgegebenen Stimulationsenergie in Form entsprechend modulierter niedrigfrequenter Stimulationsströme ausgebildet.

Die Frequenz und/oder Modulationsfrequenz der Stimulationsströme liegt im Bereich der Frequenzen, bei denen die Ausschüttung von die Zellproliferation steuernden sekundären Botenstoffen, wie dem zyklischem Adenosinmonophosphat (cAMP), in den Zellen des Gefäßes beeinflusst wird. Im gezeigten Beispiel ist nur eine Auskopplungseinheit dargestellt. Es versteht sich, dass bei anderen Varianten auch mehrere solcher Auskopplungseinheiten vorgesehen sein können, da die zur Beeinflussung erforderliche Frequenz von Zelltyp zu Zelltyp abweichen kann, und gegebenenfalls mehrere Zelltypen entsprechend stimuliert werden müssen.

Für Blutgefäße, wie das Blutgefäß 3''', liegt die Frequenz bzw. Modulationsfrequenz der Stimulationsströme zur Verhinderung bzw. Verlangsamung der Stenosenbildung bevorzugt im Bereich der Frequenzen, bei denen die Ausschüttung von die Zellproliferation steuernden sekundären Botenstoffen in den glatten Muskelzellen und zusätzlich oder alternativ in den Endothelzellen sowie zusätzlich oder alternativ in den Fibroblasten des Gefäßes beeinflusst wird, da Wucherungen dieser Zelltypen den Hauptanteil an der Stenosenbildung, insbesondere in Blutgefäßen haben. Je nach Botenstoff kann eine Anregung seiner Ausschüttung erforderlich sein, wie dies beispielsweise bei dem zyklischen Adenosinmonophosphat (cAMP) der Fall ist, es kann aber auch eine Hemmung seiner Ausschüttung erforderlich sein.

Die Intensität der durch die Erregereinrichtung 5''' erzeugten elektromagnetischen Wellen ist im gezeigten Beispiel so gewählt, dass die Stimulationsströme im zu stimulierenden Gewebe eine Stromdichte von wenigstens 5 µA/cm² erreichen.

Die Steuereinrichtung 5.4''' kann die Stimulation mit beliebigen vorgebbaren zeitlichen Stimulationsabläufen steuern. Sie weist insbesondere eine - nicht dargestellte - Zeitsteuerschaltung auf, die nach einer bestimmten vorgebbaren Stimulationsdauer zur schrittweisen Reduktion der Stimulationsintensität ausgebildet ist. Hiermit ist ein so genanntes "Ausschleichen" der Behandlung möglich, bei dem die künstliche Unterdrückung von Zellproliferationsprozessen schrittweise verringert wird, um ein überschießendes Wachstum der Zellen als Antwort auf ein abruptes Absetzen der Behandlung zu verhindern. Es versteht sich, dass sie bei anderen Varianten auch zur kontinuierlichen Reduktion der Stimulationsintensität und zusätzlich oder alternativ auch zur schrittweisen oder kontinuierlichen Reduktion der Stimulationshäufigkeit ausgebildet sein kann.

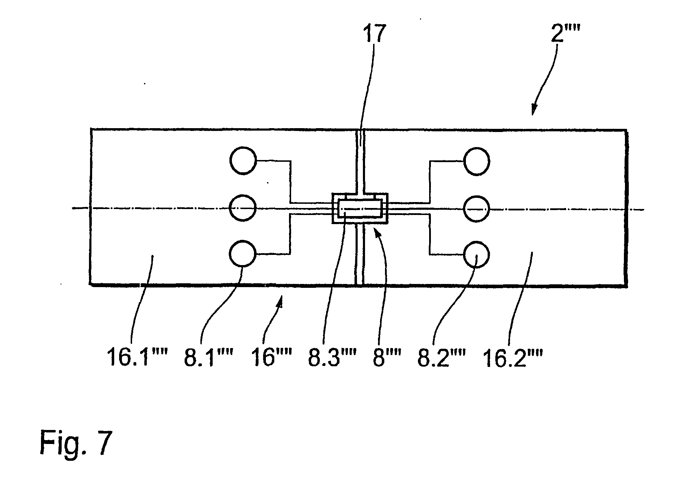

Figur 7 zeigt eine schematische Ansicht einer weiteren Ausführung eines erfindungsgemäßen Stents 2''''. Der Stent 2'''' ist dabei in der oben zu Figur 5 und 6 beschriebenen Weise zur Einkopplung von Stimulationsenergie ausgebildet, so dass hier lediglich auf seine Besonderheiten eingegangen werden soll.

Der Dipol 16'''' ist bei dieser Variante als einfacher Dipol ausgebildet. Seine Länge entspricht der halben Wellenlänge der an ihm auftreffenden, von einem entsprechenden Sender erzeugten elektromagnetischen Wellen. Hierbei stimmt die von dem Dipol 16'''' empfangene Frequenz aufgrund der Veränderung durch das Dielektrikum des Körpergewebes nicht mit der Sendefrequenz (im Vakuum) des betreffenden Senders überein. Der Dipol 16'''' besteht aus zwei elektrisch leitenden Beschichtungen 16.1'''' und 16.2'''' auf dem Grundkörper des Stents 2'''', die sich jeweils etwa über die halbe Länge des Stents 2'''' erstrecken und in der Mitte des Stents 2'''' durch eine isolierende Lage 17 voneinander getrennt sind.

Die Hälften 16.1'''' und 16.2'''' des Dipols 16'''' sind mit einer Auskopplungseinheit 8'''' in der Mitte des Stents 2'''' verbunden, welche die im Dipol 16'''' erzeugten hochfrequenten Ströme in niedrigfrequente Stimulationsströme umwandelt, die dann wiederum über eine Reihe von Elektroden 8.1'''' und 8.2'''', die gegen die Beschichtungen 16.1'''' und 16.2'''' isoliert sind, an das betreffende Blutgefäß abgegeben werden. Die Elektroden 8.1 '''' und 8.2'''' können gleichmäßig über den Stent verteilt sein, um eine gleichmäßige Stimulation des Gewebes zu erzielen.

Die zu Figur 7 beschriebene Konfiguration lässt sich mit beliebig gestalteten Grundkörpern des Stents 2'''' realisieren. So kann dieser, gegebenenfalls bis auf den kleinen Bereich, in dem die Umwandlungseinheit 8.3'''' angeordnet ist, eine beliebig gestaltete bekannte Struktur, beispielsweise eine bekannte Netzstruktur haben. Die Umwandlungseinheit kann wiederum auf einem gesonderten Pad angeordnet sein, wie dies oben zu Figur 3 beschrieben wurde.

Es versteht sich, dass bei anderen Varianten des erfindungsgemäßen Implantats, beispielsweise eines Stents, auch der Grundkörper selbst den Dipol bilden kann. Hierbei besteht dieser dann aus zwei durch einen nicht leitenden Abschnitt getrennten, elektrisch leitenden Hälften.

## Patentansprüche

1. Vorrichtung zum Beeinflussen von Zellproliferationsmechanismen in Gefäßen, insbesondere Blutgefäßen, des menschlichen oder tierischen Körpers, **dadurch gekennzeichnet, dass** zur Beeinflussung der Zellproliferationsmechanismen eine Erregereinrichtung (5; 5''') vorgesehen ist, die zur Bewirkung von Stimulationsströmen in einem zu behandelnden Bereich des Gefäßes (3; 3''') ausgebildet ist, wobei die Frequenz und/oder die Modulationsfrequenz der Stimulationsströme im Bereich der Frequenzen liegt, bei denen durch ihre biologisch wirksame Information die Ausschüttung zyklischem Adenosinmonophosphats (cAMP) in den Zellen des Gefäßes (3; 3''') gehemmt oder angeregt wird, und die Erregereinrichtung (5; 5''') zum berührungslosen Einbringen der Stimulationsenergie zur Bewirkung der Stimulationsströme in einem im zu behandelnden Bereich (3.1; 3.1''') des Gefäßes (3; 3''') angeordneten Implantat (2; 2'; 2"; 2'''; 2'''') insbesondere einem Stent, ausgebildet ist.

2. Vorrichtung nach Anspruch 1, **dadurch gekennzeichnet, dass** die Erregereinrichtung (5; 5''') zur Bewirkung von Stimulationsströmen ausgebildet ist, deren Frequenz und/oder Modulationsfrequenz im Bereich der Frequenzen liegt, bei denen die Ausschüttung von die Zellproliferation bewirkenden sekundären Botenstoffen in den glatten Muskelzellen und/oder den Endothelzellen und/oder den Fibroblasten eines Gefäßes (3; 3''') gehemmt oder angeregt wird.

3. Vorrichtung nach einem der vorhergehenden Ansprüche, **dadurch gekennzeichnet, dass** die Erregereinrichtung (5; 5''') zur Bewirkung von Stimulationsströmen in dem zu behandelnden Bereich des Gefäßes (3; 3''') mit einer Frequenz und/oder einer Modulationsfrequenz bis zu 200 Hz, vorzugsweise zwischen 10 und 100 Hz, ausgebildet ist.

4. Vorrichtung nach einem der vorhergehenden Ansprüche, **dadurch gekennzeichnet, dass** die Erregereinrichtung (5; 5''') eine Zeitssteuereinrichtung aufweist, die zur schrittweisen oder kontinuierlichen Reduktion der Stimulationsintensität und/oder der Stimulationshäufigkeit ausgebildet ist.

5. Vorrichtung nach Anspruch 1, **dadurch gekennzeichnet, dass** die Erregereinrichtung (5; 5'''*)* eine Induktionseinrichtung (5.1, 5.2, 5.3, 5.4) zur induktiven Einkopplung der Stimulationsenergie in das Implantat (2; 2'; 2") umfasst.

6. Vorrichtung nach Anspruch 1, **dadurch gekennzeichnet, dass** die Erregereinrichtung (5''') eine Sendeeinrichtung (5.9''') zur Einkopplung der Stimulationsenergie in Form elektromagnetischer Schwingungen in das Implantat (2'''; 2'''') umfasst, welches ein nach Art einer Antenne ausgebildetes Antennenelement (16; 16'''') umfasst.

7. Vorrichtung nach Anspruch 6, **dadurch gekennzeichnet, dass** eine Einrichtung (13) zum Fokussieren der elektromagnetischen Schwingungen im Bereich des Implantats (2'''; 2'''') vorgesehen ist.

8. Implantat zum Einsetzen in ein Gefäß (3; 3'''), insbesondere ein Blutgefäß, des menschlichen oder tierischen Körpers, wobei das Implantat ein Stent ist, **dadurch gekennzeichnet, dass** es zum Zellproliferationsmechanismen beeinflussenden Stimulieren von Zellen des Gefäßes (3; 3'''), in das es implantiert ist, mittels Stimulationsströmen, die von einer Erregereinrichtung (5; 5''') nach einem der Ansprüche 1 bis 7 bewirkt werden, ausgebildet ist.

9. Implantat nach Anspruch 8, **dadurch gekennzeichnet, dass** es zum Auskoppeln induktiv eingekoppelter Stimulationsenergie in Form von Stimulationsströmen ausgebildet ist.

10. Implantat nach Anspruch 9, **dadurch gekennzeichnet, dass** es einen zum Anliegen an der Wandung des Gefäßes vorgesehenen rohrförmigen Körper umfasst, der zur Ausbildung eines Resonanzkreises (7; 7") zumindest abschnittsweise als Induktionsspule ausgebildet ist.

11. Implantat nach Anspruch 8, **dadurch gekennzeichnet, dass** es zum Auskoppeln mittels elektromagnetischer Wellen eingekoppelter Stimulationsenergie in Form von Stimulationsströmen ausgebildet ist, wobei es ein nach Art einer Antenne ausgebildettes Antennenelement (16; 16'''') umfasst.

12. Implantat nach Anspruch 11, **dadurch gekennzeichnet, dass** es einen zum Anliegen an der Wandung des Gefäßes vorgesehenen rohrförmigen Körper umfasst, der nach Art einer Dipolantenne ausgebildet ist.

13. Implantat nach einem der Ansprüche 8 bis 12, **dadurch gekennzeichnet, dass** eine Auskopplungseinheit (8; 8'; 8'''; 8'''') vorgesehen ist, die eine Umwandlungseinheit (8.3; 8.3'; 8.3'''; 8.3'''') zum Umwandeln der eingekoppelten Stimulationsenergie in Stimulationsströme umfasst.

14. Implantat nach Anspruch 13, **dadurch gekennzeichnet, dass** die Umwandlungseinheit (8.3; 8.3'; 8.3'''; 8.3'''') eine elektronische Schaltung zum Umwandeln eines hochfrequenten Stromes in einen niedrigfrequenten und/oder niedrigfrequent modulierten Stimulationsstrom umfasst.

15. Implantat nach Anspruch 14, **dadurch gekennzeichnet, dass** die elektronische Schaltung in einer Beschichtung des Implantats (2; 2'; 2"; 2'''; 2'''') ausgebildet ist.

16. Implantat nach einem der Ansprüche 8 bis 15, **dadurch gekennzeichnet, dass** es zur Auskopplung von Stimulationsströmen mit einer Frequenz und/oder einer Modulationsfrequenz bis zu 200 Hz, vorzugsweise zwischen 10 und 100 Hz, ausgebildet ist.

17. Implantat einem der Ansprüche 8 bis 16, **dadurch gekennzeichnet, dass** es zur Auskopplung von Stimulationsströmen ausgebildet ist, deren Frequenz und/oder Modulationsfrequenz im Bereich der Frequenzen liegt, bei denen die Ausschüttung von die Zellproliferation steuernden sekundären Botenstoffen , insbesondere von zyklischem Adenosinmonophosphat (cAMP), in den Zellen des Gefäßes (3; 3''') beeinflusst wird.

18. Implantat nach Anspruch 17, **dadurch gekennzeichnet, dass** es zur Auskopplung von Stimulationsströmen ausgebildet ist, deren Frequenz und/oder Modulationsfrequenz im Bereich der Frequenzen liegt, bei denen die Ausschüttung von die Zellproliferation steuernden sekundären Botenstoffen in den glatten Muskelzellen und/oder den Endothelzellen und/oder den Fibroblasten des Gefäßes (3; 3''') beeinflusst wird.

19. Anordnung mit einer Stimulationsvorrichtung nach einem der Ansprüche 1 bis 7 und einem Implantat nach einem der Ansprüche 8 bis 18.

## Claims

1. An apparatus for influencing cell proliferation mechanisms in vessels, in particular blood vessels, of the human or animal body,
**characterised in that** to influence the cell proliferation mechanisms a stimulation device (5; 5'") is provided, which is designed to provoke stimulation currents in an area to be treated of the vessel (3; 3'''), the frequency and/or the modulation frequency of the stimulation currents lying in the range of the frequencies at which, by their biologically effective information, the production of cyclic adenosine monophosphate (cAMP) in the cells of the vessel (3; 3''') is inhibited or stimulated, and the stimulation device (5; 5''') for the contactless introduction of the stimulation energy for provoking the stimulation currents is constructed in an implant (2; 2'; 2"; 2'''; 2''') disposed in the area (3.1; 3.1 ''') to be treated of the vessel (3; 3'''), in particular a stent.

2. An apparatus according to Claim 1,
**characterised in that** the stimulation device (5; 5''') is constructed to provoke stimulation currents, the frequency and/or modulation frequency of which lies in the range of frequencies at which the production of secondary messengers provoking cell proliferation in the smooth muscle cells and/or the endothelial cells and/or the fibroblasts of a vessel (3; 3''') is inhibited or stimulated.

3. An apparatus according to one of the preceding Claims,
**characterised in that** the stimulation device (5; 5''') is designed to provoke stimulation currents in the area to be treated of the vessel (3; 3''') with a frequency and/or a modulation of up to 200 Hz, preferably between 10 and 100 Hz.

4. An apparatus according to one of the preceding Claims,
**characterised in that** the stimulation device (5; 5''') comprises a time control device which is designed for the gradual or continuous reduction of the stimulation intensity and/or of the stimulation frequency.

5. An apparatus according to Claim 1,
**characterised in that** the stimulation device (5; 5''') comprises an induction device (5.1, 5.2, 5.3, 5.4) for the inductive coupling of the stimulation energy into the implant (2; 2'; 2").

6. An apparatus according to Claim 1,
**characterised in that** the stimulation device (5''') comprises a transmitting device (5.9''') for coupling the stimulation energy in the form of electromagnetic oscillations into the implant (2'''; 2''''), which comprises an antenna element (16; 16'''') constructed in the manner of an antenna.

7. An apparatus according to Claim 6,
**characterised in that** a device (13) is provided for focussing the electromagnetic oscillations in the vicinity of the implant (2''', 2'''').

8. An implant for insertion into a vessel (3; 3'''), in particular a blood vessel, of the human or animal body, the implant being a stent,
**characterised in that** it is designed for the stimulation, influencing cell proliferation mechanisms, of cells of the vessel (3; 3'''), into which it is implanted by means of stimulation currents, which are produced by a stimulation device (5; 5''') according to one of Claims 1 to 7.

9. An implant according to Claim 8,
**characterised in that** it is designed to decouple inductively coupled stimulation energy in the form of stimulation currents.

10. An implant according to Claim 9,
**characterised in that** it comprises a tubular body that is provided to abut the wall of the vessel and to form a resonant circuit (7; 7") is designed at least in some sections as an induction coil.

11. An implant according to Claim 8,
**characterised in that** it is designed to decouple stimulation energy coupled by means of electromagnetic waves, it comprising an antenna element (16; 16'''') designed in the manner of an antenna.

12. An implant according to Claim 11,
**characterised in that** it comprises a tubular body which is provided to abut the wall of the vessel and is designed in the manner of a dipole antenna.

13. An implant according to one of Claims 8 to 12,
**characterised in that** a decoupling unit (8; 8'; 8'''; 8'''') is provided, which comprises a conversion unit (8.3; 8.3'; 8.3'''; 8.3'''') for converting the coupled stimulation energy into stimulation currents.

14. An implant according to Claim 13,
**characterised in that** the conversion unit (8.3; 8.3'; 8.3'''; 8.3'''') comprises an electronic circuit for converting a high-frequency current into a low-frequency and/or low-frequency-modulated stimulation current.

15. An implant according to Claim 14,
**characterised in that** the electronic circuit is constructed in a covering of the implant (2; 2'; 2", 2''', 2'''').

16. An implant according to one of Claims 8 to 15,
**characterised in that** it is constructed with a frequency and/or a modulation frequency of up to 200 Hz, preferably of between 10 and 100 Hz, for the decoupling of stimulation currents.

17. An implant according to one of Claims 8 to 16,
**characterised in that** it is constructed to decouple stimulation currents, the frequency and/or the modulation frequency of which lies in the range of frequencies at which the production of secondary messengers controlling the cell proliferation, in particular of cyclic adenosine monophosphate (cAMP), is influenced in the cells of the vessel (3; 3''').

18. An implant according to Claim 17,
**characterised in that** it is constructed for decoupling stimulation currents, the frequency and/or modulation frequency of which lies in the range of frequencies at which the production of secondary messengers controlling the cell proliferation is influenced in the smooth muscle cells and/or the endothelial cells and/or the fibroblasts of the vessel (3; 3''').

19. An arrangement having a stimulation device according to one of Claims 1 to 7 and an implant according to one of Claims 8 to 18.

## Revendications

1. Dispositif pour accélérer la prolifération de cellules dans des vaisseaux, en particulier des vaisseaux sanguins, du corps humain ou animal, **caractérisé en ce que**, pour accélérer la prolifération de cellules, un mécanisme d'excitation (5 ; 5''') est prévu, qui est réalisé pour provoquer des courants de stimulation dans une zone à traiter du vaisseau (3 ; 3'''), où la fréquence et/ou la fréquence de modulation des courants de stimulation se trouve dans une plage de fréquences, dans lesquelles, grâce à leur information active biologiquement, la sécrétion de monophosphate d'adénosine cyclique (cAMP) dans les cellules du vaisseau (3 ; 3''') est bloquée ou déclenchée, et le mécanisme d'excitation (5 ; 5''') est réalisé en vue de l'introduction sans contact de l'énergie de stimulation pour provoquer des courants de stimulation dans un implant (2 ; 2' ; 2''' ; 2''''), en particulier un stent, disposé dans la zone à traiter (3.1 ; 3.1''') du vaisseau (3 ; 3''').

2. Dispositif selon la revendication 1, **caractérisé en ce que** le mécanisme d'excitation (5 ; 5''') est réalisé pour provoquer des courants de stimulation, dont la fréquence et/ou la fréquence de modulation se trouve dans une plage de fréquences, dans lesquelles la sécrétion de tissus messagers secondaires provoquant la prolifération de cellules dans les cellules musculaires lisses et/ou les cellules endothéliales et/ou les fibroblastes d'un vaisseau (3 ; 3''') est bloquée ou déclenchée.

3. Dispositif selon l'une des revendications précédentes, **caractérisé en ce que** le mécanisme d'excitation (5 ; 5''') est réalisé avec une fréquence et/ou une fréquence de modulation allant jusqu'à 200 Hz, de préférence entre 10 et 100 Hz, pour provoquer des courants de stimulation dans la zone à traiter du vaisseau (3 ; 3''').

4. Dispositif selon l'une des revendications précédentes, **caractérisé en ce que** le mécanisme d'excitation (5 ; 5''') présente un mécanisme de synchronisation, qui est réalisé en vue de la réduction progressive ou continue de l'intensité de stimulation et/ou de la fréquence de stimulation.

5. Dispositif selon la revendication 1, **caractérisé en ce que** le mécanisme d'excitation (5 ; 5''') comprend un mécanisme d'induction (5.1, 5.2, 5.3, 5.4) en vue du couplage inductif de l'énergie de stimulation dans l'implant (2 ; 2' ; 2").

6. Dispositif selon la revendication 1, **caractérisé en ce que** le mécanisme d'excitation (5''') comprend un mécanisme émetteur (5.9''') en vue du couplage de l'énergie de stimulation sous la forme d'oscillations électromagnétiques dans l'implant (2''' ; 2''''), qui comprend un élément d'antenne (16 ; 16'''') réalisé à la manière d'une antenne.

7. Dispositif selon la revendication 6, **caractérisé en ce qu'**un mécanisme (13) est prévu pour concentrer les oscillations électromagnétiques dans la zone de l'implant (2''' ; 2'''').

8. Implant en vue d'une insertion dans un vaisseau (3 ; 3'''), en particulier un vaisseau sanguin, du corps humain ou animal, où l'implant est un stent, **caractérisé en ce qu'**il est réalisé pour la stimulation accélérant la prolifération de cellules des cellules du vaisseau (3 ; 3'''), dans lequel il est implanté, au moyen de courants de stimulation, qui sont générés par un mécanisme d'excitation (5 ; 5''') selon l'une des revendications 1 à 7.

9. Implant selon la revendication 8, **caractérisé en ce qu'**il est réalisé en vue du découplage de l'énergie de stimulation couplée de façon inductive sous la forme de courants de stimulation.

10. Implant selon la revendication 9, **caractérisé en ce qu'**il comprend un élément tubulaire prévu pour adhérer à la paroi d'un vaisseau, qui est réalisé en vue de la formation d'un circuit résonnant (7 ; 7") au moins par tronçon en tant que bobine d'induction.

11. Implant selon la revendication 8, **caractérisé en ce qu'**il est réalisé en vue du découplage de l'énergie de stimulation couplée au moyen d'ondes électromagnétiques sous la forme de courants de stimulation, où il comprend un élément d'antenne (16 ; 16'''') réalisé à la manière d'une antenne.

12. Implant selon la revendication 11, **caractérisé en ce qu'**il comprend un élément tubulaire prévu pour adhérer à la paroi d'un vaisseau, qui est réalisé à la manière d'un dipôle.

13. Implant selon l'une des revendications 8 à 12, **caractérisé en ce qu'**une unité de découplage (8 ; 8' ; 8''' ; 8'''') est prévue, qui comprend une unité de conversion (8.3 ; 8.3' ; 8.3''' ; 8.3'''') en vue de la conversion de l'énergie de stimulation couplée dans les courants de stimulation.

14. Implant selon la revendication 13, **caractérisé en ce que** l'unité de conversion (8.3 ; 8.3' ; 8.3''' ; 8.3'''') comprend un circuit électronique en vue de la conversion d'un courant à haute fréquence en un courant de stimulation à une basse fréquence et/ou modulé en basse fréquence.

15. Implant selon la revendication 14, **caractérisé en ce que** le circuit électronique est réalisé dans un revêtement de l'implant (2 ; 2' ; 2" ; 2''' ; 2'''').

16. Implant selon l'une des revendications 8 à 15, **caractérisé en ce qu'**il est réalisé en vue du déclenchement de courants de stimulation avec une fréquence et/ou une fréquence de modulation allant jusqu'à 200 Hz, de préférence entre 10 et 100 Hz.

17. Implant selon l'une des revendications 8 à 16, **caractérisé en ce qu'**il est réalisé en vue du découplage de courants de stimulation, dont la fréquence et/ou la fréquence de modulation se trouve dans une plage de fréquences, dans lesquelles la sécrétion de tissus messagers secondaires, en particulier le monophosphate d'adénosine (cAMP), déclenchant la prolifération de cellules dans les cellules du vaisseau (3 ; 3'''), est accélérée.

18. Implant selon la revendication 17, **caractérisé en ce qu'**il est réalisé en vue du découplage de courants de stimulation, dont la fréquence et/ou la fréquence de modulation se trouve dans une plage de fréquences, dans lesquelles la sécrétion de tissus messagers secondaires déclenchant la prolifération de cellules dans les cellules de muscles lisses et/ou les cellules endothéliales et/ou les fibroblastes du vaisseau (3 ; 3''') est accélérée.

19. Agencement avec un dispositif de stimulation selon l'une des revendications 1 à 7 et un implant selon l'une des revendications 8 à 18.
